# EUROPÄISCHE PATENTSCHRIFT

(11) **EP 1 341 454 B1**
(45) Veröffentlichungstag und Bekanntmachung des Hinweises auf die Patenterteilung: **02.09.2009**
(21) Anmeldenummer: 01995596.2
(22) Anmeldetag: 12.12.2001
(51) Int. Cl.: A61B 17/22

(54) **KOPPELBALG ZUR STOSSWELLENTHERAPIE**
COUPLING BELLOWS FOR SHOCKWAVE THERAPY
SOUFFLET DE COUPLAGE POUR THERAPIE A ONDES DE CHOC

(30) Priorität: 15.12.2000 DE 10062749
(43) Veröffentlichungstag der Anmeldung: 10.09.2003
(73) Patentinhaber: Dornier MedTech Holding International GmbH, 82234 Wessling (DE)
(72) Erfinder: EIZENHÖFER, Harald, 82229 Seefeld (DE)
(74) Vertreter: Grünecker, Kinkeldey, Stockmair & Schwanhäusser Anwaltssozietät
(86) Internationale Anmeldenummer: PCT/DE2001/004710
(87) Internationale Veröffentlichungsnummer: WO 2002/047560

(56) Entgegenhaltungen:
- EP-A- 0 445 322
- DE-A- 19 625 164
- DE-C- 19 509 004
- DE-U- 9 102 394
- DE-U- 29 712 035
- US-A- 4 798 196

## Beschreibung

Die Erfindung bezieht sich auf einen Koppelbalg, wie er beispielsweise aus dem DE GBM 29712035 bekannt ist und verwendet wird, um eine Stoßwellenquelle an einen Patientenkörper zu koppeln.

Bei der Therapie mit Stoßwellen entsteht mit jeder Stoßwelle auch störender Luftschall, der subjektiv als lauter "Schuss" wahrgenommen wird. Diese Lärmquelle wird vom Patienten als auch vom behandelnden Personal als Belastung empfunden.

Dieser störende Luftschall wird hauptsächlich von dem Bereich des Koppelbalgs abgestrahlt, der nicht durch den Körper des Patienten abgedeckt ist. Stand der Technik ist es, an der Mantelflache des Koppelbalgs zusätzliches, lärmdämmendes Material anzubringen wie z.B. Handtücher, Sandsäckchen oder speziell entworfene Lärmschutzmanschetten.

Die bekannten Lösungen haben nun mehrere Nachteile. Zum einen sind sie unhandlich und/oder schwer, zum anderen sind sie bei der Ortung des Konkrements hinderlich, da sie in den Strahlengang des Röntgengerätes oder des Ultraschallgerätes hineinreichen.

Aus der EP 0 445 322 A1 ist eine Koppelbalganordnung für einen Lithotripter bekannt. Die Koppelbalganordnung weist ein Bodenelement und ein Seitenelement auf. Das Seitenelement ist als ein flexibler Faltenbalg ausgebildet.

Es ist Aufgabe der Erfindung, einen Koppelbalg zu schaffen, bei dem weniger Luftschall abgestrahlt wird, ohne dass die Ortung des Konkrements eingeschränkt wird.

Diese Aufgabe wird durch die kennzeichnenden Merkmale von Anspruch 1 gelöst. Die Unteransprüche betreffen Weiterbildungen und/oder besonders vorteilhafte Ausgestaltungen der Ereindung.

Nachfolgend ist die Erfindung anhand einer Figur näher erläutert.

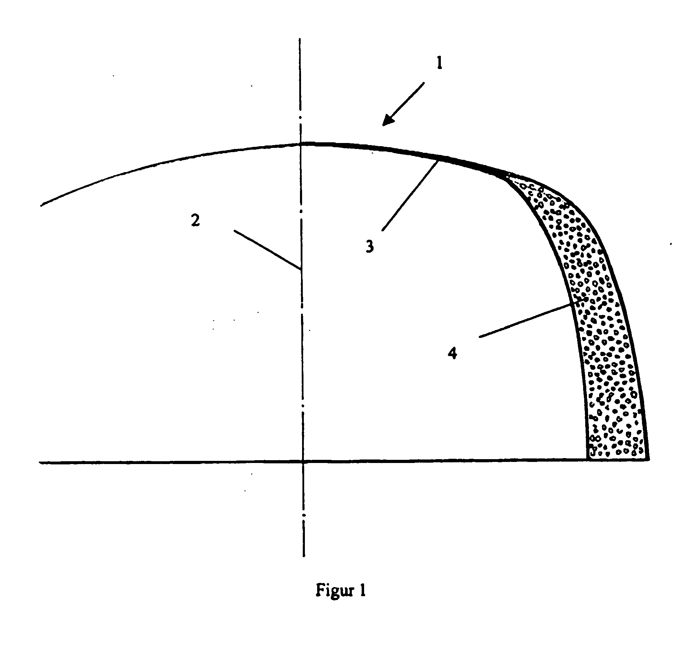

Die Figur 1 zeigt den Koppelbalg 1 in geschnittener Darstellung. Sein Aufbau ist im wesentlichen rotationssymmetrisch zur Achse 2.
Der mit dem Patienten in Berührung kommende Koppelbereich 3 ist dünnwandig ausgebildet und hat keine Einschlüsse. Die Stosswellen werden hierdurch nur minimal absorbiert.

Der seitliche Mantelbereich 4 hingegen ist durch Luftporen oder andere Füllstoffe auf maximale Schallabsorption ausgelegt.

Die Luftporen haben weiterhin den Vorteil, dass der Balg dadurch elastischer wird und sich den verschiedenen Eindringtiefen, die jeweils eine andere Ausdehnung des Koppelbalges (entlang der Achse 2) erfordern, besser anpassen kann als ein homogener Werkstoff ohne Poren.

Idealerweise wird der erfindungsgemässe Koppelbalg im sog. "compression molding"-Verfahren hergestellt. Hierbei werden in ein Werkzeug eine oder mehrere, zugeschnittene, teilvulkanisierte Matten eingelegt.
Unter Druck- und Temperaturbeaufschlagung werden die Zuschnitte pastös bis flüssig, verbinden sich miteinander und vulkanisieren vollständig aus.

Erfindungsgemäss wird für den Mantelbereich 4 ein Werkstoff ausgewählt, der bei der Vulkanisation/Polymerisation Poren entwickelt ("Moosgummi"), während das Einlegeteil für den Koppelbereich 3 optimale Stosswellen- und Ultraschalltransmission aufweist. Als Werkstoffe eignen sich nahezu alle Elastomere wie die grosse Klasse der Gummiwerkstoffe; daneben auch die Silikone und die Polyurethane.

## Patentansprüche

1. Koppelbalg zur Kopplung einer Stoßwellenquelle an einen Patientenkörper, der mit einer Flüssigkeit oder einem Gel befüllbar ist, der
- im Wesentlichen rotationssymmetrisch ausgebildet ist,
- einen im Wesentlichen senkrecht zur Rotationsachse verlaufenden als Koppelfläche zum Patientenkörper ausgebildeten Bereich und
- einen im Wesentlichen zylindrisch zur Rotationsachse verlaufenden Mantelbereich aufweist,
wobei der Koppelbereich (3) transmissiv für die Stoßwellen ausgebildet ist,
**dadurch gekennzeichnet, dass**
der Mantelbereich (4) mit Luftporen und/oder sonstigen schalldämmenden Füllstoffen versehen schalldämmend ausgebildet ist.

2. Koppelbalg nach Anspruch 1,
**dadurch gekennzeichnet, dass**
der Koppelbereich (3) dünnwandig und frei von Einschlüssen, der Mantelbereich (4) dickwandiger ausgebildet ist.

3. Koppelbalg nach Anspruch 1 oder 2,
**dadurch gekennzeichnet, dass**
der Koppelbereich (3) und der Mantelbereich (4) aus unterschiedlichen, jedoch flüssigkeitsdicht miteinander verbundenen Materialien bestehen.

4. Koppelbalg nach Anspruch 1 bis 3,
**dadurch gekennzeichnet, dass**
das für den Koppelbereich (3) und den Mantelbereich (4) verwendete Material vulkanisierbar ist.

5. Koppelbalg nach Anspruch 4,
**dadurch gekennzeichnet, dass**
das für den Mantelbereich (4) verwendete Material nach der Vulkanisation eine poröse Struktur aufweist.

## Claims

1. Coupling bellows for coupling a shock wave source to a patient's body, which bellows may be filled with a fluid or a gel and which
- are of substantially rotationally symmetrical construction,
- comprise a region extending substantially perpendicularly to the axis of rotation and taking the form of a coupling surface for coupling to the patient's body and
- comprise an outer circumferential region extending substantially cylindrically relative to the axis of rotation,
the coupling region (3) being transmissive to shock waves,
**characterised in that**
the outer circumferential region (4) is of sound-absorbing construction, being provided with air voids and/or other sound-absorbing fillers.

2. Coupling bellows according to claim 1,
**characterised in that**
the coupling region (3) is thin-walled and free of inclusions, while the outer circumferential region (4) is of thicker-walled construction.

3. Coupling bellows according to claim 1 or claim 2,
**characterised in that**
the coupling region (3) and the outer circumferential region (4) consist of different materials connected however in a fluid-tight manner to one another.

4. Coupling bellows according to claims 1 to 3,
**characterised in that**
the material used for the coupling region (3) and the outer circumferential region (4) is vulcanisable.

5. Coupling bellows according to claim 4,
**characterised in that**
the material used for the outer circumferential region (4) has a porous structure after vulcanisation.

## Revendications

1. Soufflet de couplage destiné à accoupler une source d'ondes de choc au corps d'un patient, lequel soufflet peut être rempli avec un liquide ou un gel, lequel
- est réalisé sensiblement selon une symétrie de révolution,
- comporte une zone réalisée sous forme de surface de couplage avec le corps du patient, orientée sensiblement perpendiculairement à l'axe de rotation, et
- une zone d'enveloppe s'étendant sensiblement sous forme cylindrique par rapport à l'axe de rotation,
ladite zone de couplage (3) étant réalisée sous forme de zone transmissive pour les ondes de choc
**caractérisé en ce que**
la zone d'enveloppe (4), réalisée sous forme de zone insonorisante, est munie de pores d'air et/ou autres matériaux de remplissage insonorisants.

2. Soufflet de couplage selon la revendication 1, **caractérisé en ce que** la zone de couplage (3) est réalisée avec une paroi de faible épaisseur et ne contient pas d'inclusions, la zone d'enveloppe (4) étant réalisée avec une paroi plus épaisse.

3. Soufflet de couplage selon la revendication 1 ou 2, **caractérisé en ce que** la zone de couplage (3) et la zone d'enveloppe (4) sont réalisées dans des matériaux différents, mais reliés entre eux de manière étanche aux liquides.

4. Soufflet de couplage selon l'une quelconque des revendications 1 à 3, **caractérisé en ce que** le matériau utilisé pour la zone de couplage (3) et la zone d'enveloppe (4) est un matériau apte à être vulcanisé.

5. Soufflet de couplage selon la revendication 4, **caractérisé en ce que** le matériau utilisé pour la zone d'enveloppe (4) possède une structure poreuse après la vulcanisation.
